# EUROPEAN PATENT APPLICATION

(11) **EP 3 255 077 A1**
(43) Date of publication of application: **13.12.2017**
(21) Application number: 16746693.7
(22) Date of filing: 04.02.2016
(51) Int. Cl.: C08G 59/12, C08G 59/24

(54) **ESTER-TYPE EPOXY FURAN RESIN AND MANUFACTURING METHOD THEREFOR, RESIN COMPOSITION, AND CURED RESIN PRODUCT**

(30) Priority: 05.02.2015 JP 2015021147
(71) Applicant: Sekisui Chemical Co., Ltd., Osaka-shi, Osaka 530-8565 (JP)
(72) Inventor: EGUCHI, Yuji, Tsukuba-shi Ibaraki 300-4292 (JP)
(74) Representative: Merkle, Gebhard
(86) International application number: PCT/JP2016/053380
(87) International publication number: WO 2016/125862

(57) **Abstract**

The ester-type epoxy furan resin of the present invention is represented by the following general formula (1). 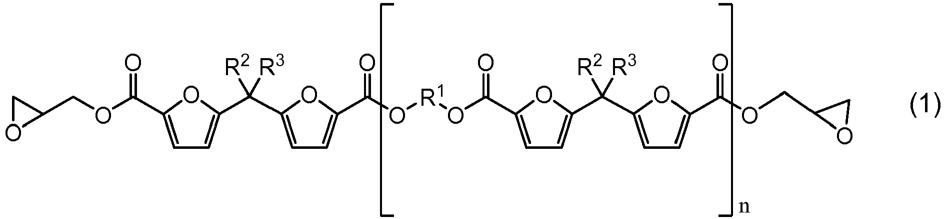 (In the general formula (1), R¹ is an alkylene group having 2 to 6 carbon atoms optionally containing a hydroxy group; R² and R³ are each an alkyl group having 1 to 4 carbon atoms, and R² and R³ may be bonded to each other to form a cyclic structure; n is 0 to 300; and a plurality of R¹, R², and R³, if present within the molecule, may be mutually the same or different.)

## Description

### Technical Field

The present invention relates to an ester-type epoxy furan resin and a method for producing the ester-type epoxy furan resin and, moreover, a resin composition and a cured resin product containing the ester-type epoxy furan resin.

### Background Art

Epoxy resins are superior in mechanical strength, heat resistance, chemical resistance, adhesion, and the like as well as have small cure shrinkage and the like, and are thus widely used in a variety of fields. Most of the epoxy resins have an aromatic cyclic structure, and petroleum-derived raw materials are commonly used therefor. On the other hand, in recent years, there are concerns about the depletion of petroleum resources, and research is conducted on the use of renewable resources such as plants for various materials. For epoxy resins, to date, research has been conducted on the use of lignin, which is contained in large amounts in lignified plants such as wood, bamboo, straw, and rice (for example, see PTL1).

### Citation List

### Patent Literature

PTL1: JP 2012-236811 A

### Summary of Invention

### Technical Problem

However, since lignin-derived epoxy resins are typically mixtures having complex structures, it is difficult to control reactions, and it can be difficult to produce such a resin having a desired structure. Accordingly, it can be difficult to stably produce a cured resin product having superior physical properties such as mechanical strength and extensibility.

On the other hand, research is increasingly conducted on the development of resins in which furan compounds are used, because furan compounds such as furfural and hydroxymethylfurfural can be obtained from renewable resources such as agricultural wastes and, also, it is relatively easy to control the structure.

The present invention has been conceived in view of the above circumstances, and an object of the present invention is to provide an epoxy resin, the reaction for which is easily controlled although a furan compound derivable from a renewable resource is used as a raw material, and which can improve physical properties such as mechanical strength and extensibility of a cured resin product.

### Solution to Problem

As a result of having conducted diligent research, the inventors found that the above problems can be solved by an ester-type epoxy furan resin having a specific structure, and accomplished the following invention. The present invention provides the following items [1] to [10].
[1] An ester-type epoxy furan resin represented by the following general formula (1): 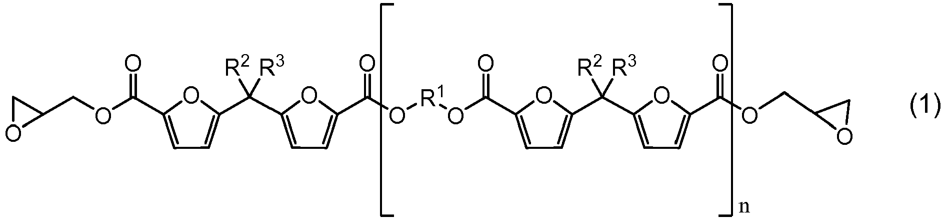 wherein R¹ is an alkylene group having 2 to 6 carbon atoms optionally containing a hydroxy group; R² and R³ are each independently an alkyl group having 1 to 4 carbon atoms, and R² and R³ may be bonded to each other to form a cyclic structure; n is 0 to 300; and a plurality of R¹, R², and R³, if present within a molecule, may be mutually the same or different.
[2] The ester-type epoxy furan resin according to the item [1], represented by the following general formula (2): 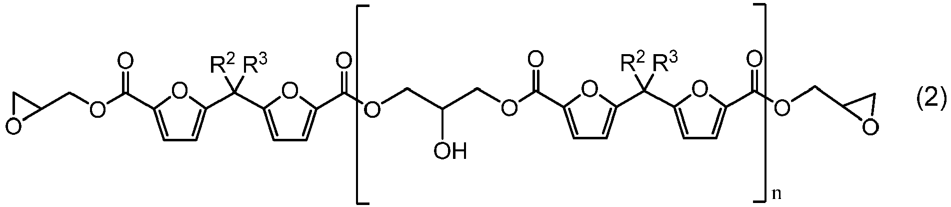 wherein R², R³, and n are the same as above.
[3] The ester-type epoxy furan resin according to the item [2], represented by the following general formula (3): 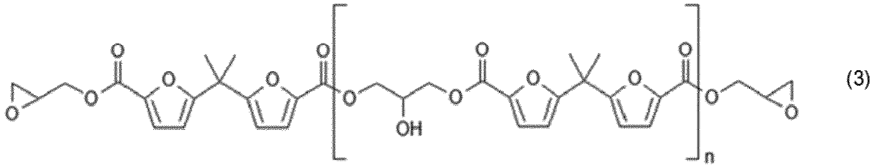 wherein n is the same as above.
[4] The ester-type epoxy furan resin according to any one of the items [1] to [3], derived from a renewable resource.
[5] A method for producing the ester-type epoxy furan resin according to any one of the items [1] to [4], comprising reacting a dicarboxylic acid compound represented by the following general formula (4) with epihalohydrin: 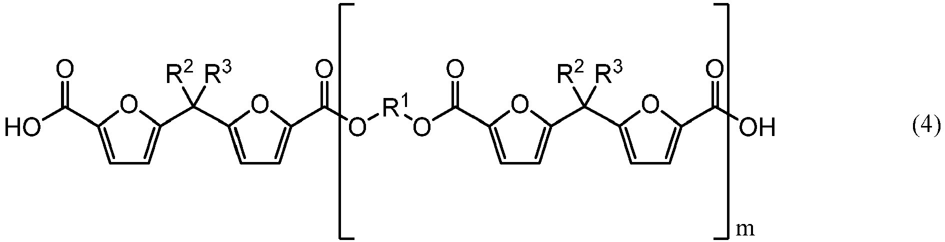 wherein R¹, R², and R³ are the same as above; and m is 0 to 300.
[6] A method for producing the ester-type epoxy furan resin according to any one of the items [1] to [4], comprising:
   reacting an ester-type epoxy furan resin having a structure represented by the general formula (1) wherein n is less than that of an ester-type epoxy furan resin to be obtained with a dicarboxylic acid compound represented by the following general formula (4-1), to obtain an ester-type epoxy furan resin represented by the general formula (1): 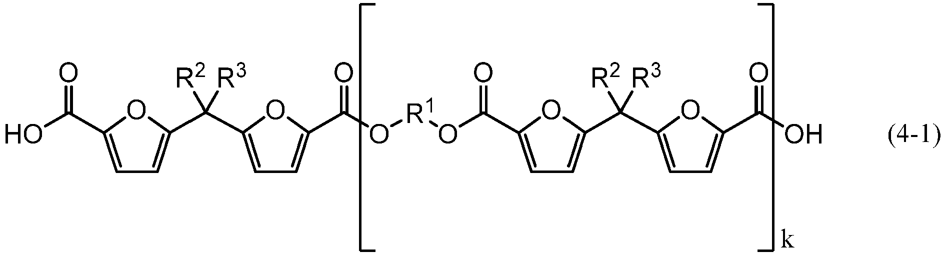
   wherein R¹, R², and R³ are the same as above; and k is 0 or more and less than 300.
[7] The method for producing an ester-type epoxy furan resin according to the item [5] or [6], wherein the dicarboxylic acid compound is a bisfurandicarboxylic acid compound represented by the following general formula (5): 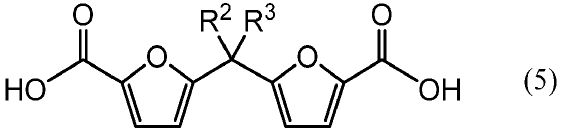 wherein R² and R³ are the same as above.
[8] The method for producing an ester-type epoxy furan resin according to any one of the items [5] to [7], wherein the dicarboxylic acid compound is derived from a renewable resource.
[9] A resin composition comprising the ester-type epoxy furan resin according to any one of the items [1] to [4] and at least any of an epoxy curing agent and a resin other than the ester-type epoxy furan resin.
[10] A cured resin product, obtained by curing the resin composition according to the item [9].

### Advantageous Effects of Invention

The present invention can provide an epoxy resin, the reaction for which is easily controlled, and which can improve physical properties such as mechanical strength and extensibility of a cured resin product.

### Description of Embodiments

Below, the present invention is described in more detail with reference to embodiments.

The ester-type epoxy furan resin of the present invention (hereinafter also simply referred to as a "furan resin") has the structure of the following general formula (1): 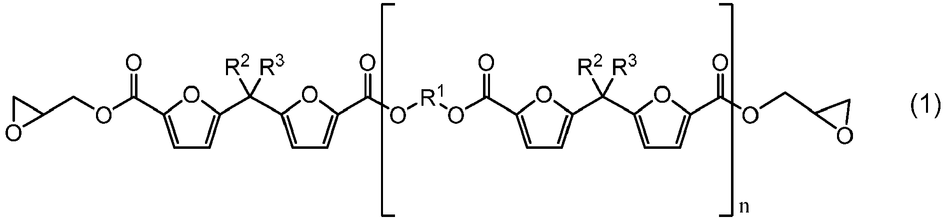 wherein R¹ is an alkylene group having 2 to 6 carbon atoms optionally containing a hydroxy group; R² and R³ are each independently an alkyl group having 1 to 4 carbon atoms, and R² and R³ may be bonded to each other to form a cyclic structure; n is 0 to 300; and a plurality of R¹, R², and R³, if present within a molecule, may be mutually the same or different.

Examples of R¹ include alkylene groups such as an ethylene group, a methylethylene group (a propylene group), a trimethylene group, an ethylethylene group, a dimethylethylene group, a methyltrimethylene group, a dimethyltrimethylene group, a tetramethylene group, a pentamethylene group, and a hexamethylene group; and alkylene groups containing one hydroxy group, such as a 2-hydroxytrimethylene group.

Examples of R² and R³ include a methyl group, an ethyl group, a n-propyl group, an isopropyl group, a n-butyl group, an isobutyl group, a tert-butyl group, and a sec-butyl group. When R² and R³ are bonded to form a cyclic structure, the total number of carbon atoms of R² and R³ is 2 to 8, and specific examples of the cyclic structure include cyclopentane and cyclohexane.

In the general formula (1), R¹ is preferably an alkylene group having 2 to 4 carbon atoms optionally containing one hydroxy group, and is more preferably a 2-hydroxytrimethylene group.

That is to say, the furan resin is more suitably a compound represented by the following general formula (2). As will be described below, the compound represented by the general formula (2) can be obtained through a one-step reaction between a bisfurandicarboxylic acid compound and epihalohydrin, and the production thereof is thus easy. A cured resin product obtained from the compound represented by the general formula (2) is likely to have better mechanical strength, extensibility, and the like, and, moreover, the hydroxy group contained therein is likely to impart good adhesion.

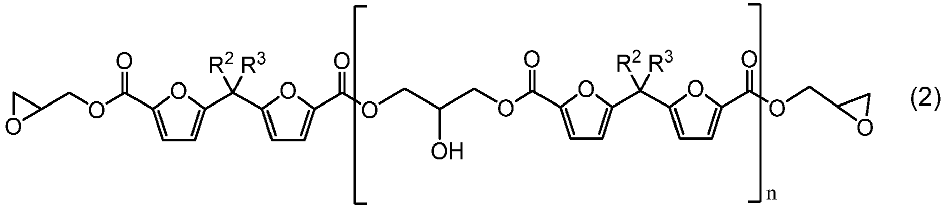

In the general formula (2), R², R³, and n are the same as above.

From the viewpoint of producibility and the like, R² and R³ in the general formulae (1) and (2) are preferably each independently an alkyl group having 1 to 2 carbon atoms, such as a methyl group or an ethyl group, and more preferably a methyl group. Accordingly, the compound represented by the general formula (2) is more preferably a compound represented by the following general formula (3).

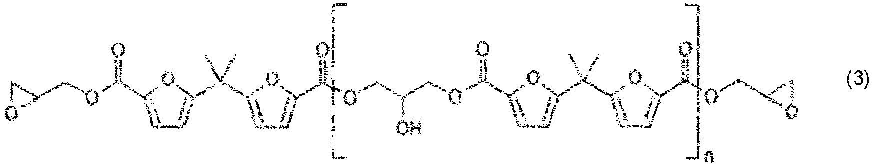

In the general formula (3), n is the same as above.

In the compound represented by the general formulae (1) to (3), n is 0 to 300. From the viewpoint of improving the reactivity of the furan resin and the physical properties of the cured product, n is preferably 0 to 50, more preferably 0 to 20, and even more preferably 0 to 10.

The weight average molecular weight (Mw) of the furan resin is generally 120000 or less, preferably 376 to 20000, more preferably 400 to 8000, and even more preferably 450 to 4000. With a furan resin having a low weight average molecular weight (Mw), the cured product of the furan resin is likely to have improved various physical properties, in particular, mechanical strength. The weight average molecular weight (Mw) herein is obtained in terms of standard polystyrene by the GPC method as presented in the examples described below.

### [Method for producing furan resin]

The method for producing a furan resin of the present invention is a production method comprising reacting a dicarboxylic acid compound represented by the following general formula (4) with epihalohydrin: 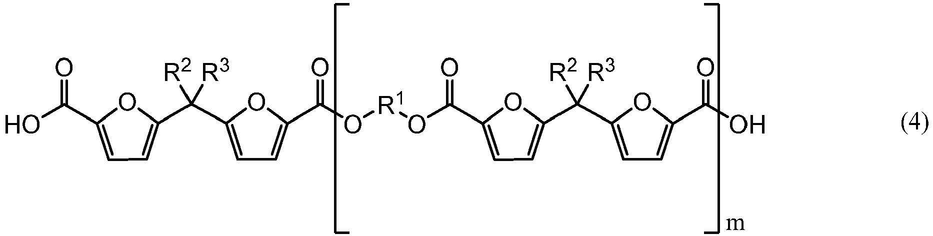 wherein R¹, R², and R³ are the same as above; and m is 0 to 300.

Here, the specific description of R¹, R², and R³ in the general formula (4) is the same as above and thus omitted.

In the present production method, as will be described below, when the above dicarboxylic acid compound and an excessive amount of epihalohydrin are reacted, normally a dicarboxylic acid compound with a glycidyl group bonded to both terminals is generated and polymerized so that the number of repeating units thereof is increased. Accordingly, m in the raw-material dicarboxylic acid compound represented by the general formula (4) is preferably less than n in the intended-product furan resin represented by the general formulae (1) to (3).

Specifically, m in the general formula (4) is preferably 0 to 150, more preferably 0 to 25, even more preferably 0 to 10, and most preferably 0 to 5.

The dicarboxylic acid compound can be produced from biomass, or that is to say, the above furan resin and dicarboxylic acid compound are, normally, derived from renewable resources.

As for the present production method, a bisfurandicarboxylic acid compound represented by the general formula (4) wherein m is 0 is preferably used as the dicarboxylic acid compound.

That is to say, a suitable specific example of the production method of the present invention is a method comprising reacting a dicarboxylic acid compound (a bisfurandicarboxylic acid compound) represented by the following general formula (5) with epihalohydrin to produce a furan resin represented by the above general formula (2) or (3) (hereinafter also referred to as production method 1). According to production method 1, it is easy to produce the furan resin because the furan resin can be obtained through a one-step reaction between a bisfurandicarboxylic acid compound and epihalohydrin.

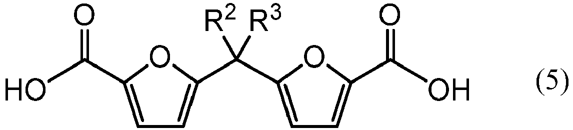

In the general formula (5), R² and R³ are the same as above.

Examples of epihalohydrin used in the present production method include epifluorohydrin, epichlorohydrin, epibromohydrine, and epiiodohydrin. Among these, epichlorohydrin is preferable in terms of reactivity and economy.

In the above production method 1, an excessive amount of epihalohydrin is mixed with the dicarboxylic acid compound to react these, and epihalohydrin is preferably 2.2 to 100 mol relative to 1 mol of the dicarboxylic acid compound. In the present production method, a small amount of epihalohydrin results in an increased molecular weight of the furan resin. Accordingly, as described above, in order for the furan resin to have a low molecular weight, epihalohydrin is preferably 2.5 to 50 mol and more preferably 5 to 30 mol relative to 1 mol of the dicarboxylic acid compound.

The reaction in the above production method 1 is normally carried out in the presence of a catalyst and an alkali compound. Examples of the catalyst include quaternary ammonium salts and the like, such as benzyltriethylammonium chloride, benzyltriethylammonium bromide, tetra-n-butylammonium fluoride, and tetra-n-butylammonium bromide. The amount of the catalyst used is preferably 0.01 to 1 mol and more preferably 0.02 to 0.2 mol relative to 1 mol of the dicarboxylic acid compound.

Examples of the alkali compound include alkali metal hydroxides such as sodium hydroxide and potassium hydroxide, and alkali metal carbonates such as sodium carbonate and potassium carbonate, and the amount of the alkali compound used is preferably 1.5 to 8 mol and more preferably 2 to 4 mol relative to 1 mol of the dicarboxylic acid compound.

In the above production method 1, both the catalyst and the alkali compound may be present in the reaction system (in a mixture of epihalohydrin and the dicarboxylic acid compound) from the beginning of the reaction. It is also possible that only the catalyst is present in the reaction system at the beginning of the reaction and, further, the alkali compound is added to the reaction system after the reaction has progressed to some extent. The present reaction is carried out at, for example, 20 to 200°C and preferably 50 to 150°C, for example, 30 minutes to 6 hours and preferably 1 to 5 hours.

Note that, in the method for producing a furan resin of the present invention, a dicarboxylic acid ester compound of the general formula (4) wherein m is 1 or more may be used as the dicarboxylic acid compound.

That is to say, another specific example of the method for producing a furan resin of the present invention is a method comprising reacting a dicarboxylic acid ester compound represented by the general formula (4) wherein m is 1 to 300 with epihalohydrin to obtain the compound represented by the above general formula (1) (hereinafter also referred to as production method 2).

From the viewpoint of the producibility and the like of the dicarboxylic acid ester compound used in production method 2, R¹ in the general formula (4) is preferably an alkylene group having 2 to 6 carbon atoms and more preferably an alkylene group having 2 to 4 carbon atoms. In production method 2, m is preferably 1 to 150, more preferably 1 to 25, even more preferably 1 to 10, and most preferably 1 to 5.

The dicarboxylic acid ester compound used in production method 2 (i.e., the compound represented by the general formula (4) wherein m is 1 to 300) is obtained by a known polyester production method, and, for example, is obtained by reacting the bisfurandicarboxylic acid compound represented by the above general formula (5) with a polyalcohol compound such as a diol compound.

Here, examples of the diol compound used include various diols having 1 to 6 carbon atoms, such as ethylene glycol, propylene glycol, 1,3-propanediol, 1,2-butanediol, 1,3-butanediol, 1,4-butanediol, 2,3-butanediol, 2-methyl-1,3-propanediol, neopentyl glycol, 1,5-pentanediol, and 1,6-hexanediol.

In production method 2, 2 mol or more of epihalohydrin is mixed with 1 mol of the dicarboxylic acid compound to react these, and preferably 2.2 to 100 mol, more preferably 2.5 to 50 mol, and even more preferably 5 to 30 mol of epihalohydrin is used relative to 1 mol of the dicarboxylic acid compound. Other conditions of production method 2 are also the same as production method 1, and the description thereof is thus omitted.

### (Chain-extending reaction)

The ester-type epoxy furan resin represented by the above general formula (1) can also be obtained by a chain-extending reaction. Specifically, an ester-type epoxy furan resin (a starting material) having a structure represented by the general formula (1) wherein n is less than that of an ester-type epoxy furan resin to be obtained may be further reacted with a dicarboxylic acid compound represented by the following general formula (4-1) to obtain an ester-type epoxy furan resin represented by the above general formula (1). The present chain-extending reaction makes it possible to easily polymerize the ester-type epoxy furan resin.

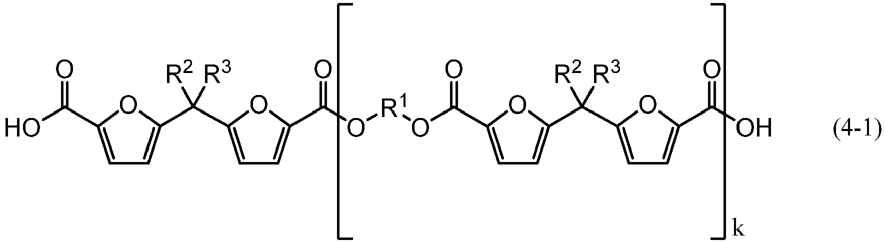

In the general formula (4-1), R¹, R², and R³ are the same as above; and k is 0 or more and less than 300, preferably 0 to 100, more preferably 0 to 10, even more preferably 0 to 5, and particularly preferably 0.

In this reaction, preferably 0.1 to 1.5 mol and more preferably 0.5 to 0.99 mol of the dicarboxylic acid compound is mixed with 1 mol of the ester-type epoxy furan resin (the starting material) to react these. When the molar number of the dicarboxylic acid compound is less than the molar number of the starting material, an epoxy group is likely to remain at both terminals, and the intended product is likely to be obtained at high purity. In addition, a larger molecular weight can be attained by setting the molar number of the dicarboxylic acid compound less than, but close to, the molar number of the starting material. The molar number of the starting material is based on the number average molecular weight by the GPC measurement.

In this chain-extending reaction as well, the bisfurandicarboxylic acid compound represented by the general formula (5) is particularly preferably used as the dicarboxylic acid compound, and the ester-type epoxy furan resin to be obtained is preferably the compound represented by the general formula (2) and even more preferably the compound represented by the general formula (3).

The ester-type epoxy furan resin as a starting material can be obtained by reacting the dicarboxylic acid compound represented by the general formula (4) with epihalohydrin as described above. The weight average molecular weight (Mw) of the starting-material, ester-type epoxy furan resin is preferably 376 to 8000, more preferable 376 to 6000, and even more preferably 376 to 3000.

The above chain-extending reaction (the reaction between the ester-type epoxy furan resin and the dicarboxylic acid compound) may be carried out in the presence of a solvent.

The solvent is not particularly limited as long as it does not inhibit the reaction, and examples include ketones such as acetone, methyl ethyl ketone, and methyl isobutyl ketone; amides such as N,N-dimethylformamide, N,N-dimethylacetamide, and N-methylpyrrolidone; ethers such as diethyl ether, diisopropyl ether, tetrahydrofuran, dimethoxyethane, and dioxane; aromatic hydrocarbons such as benzene, toluene, and xylene; halogenated aromatic hydrocarbons such as chlorobenzene and dichlorobenzene; and halogenated aliphatic hydrocarbons such as dichloromethane and chloroform. One of these solvents may be used singly or as a combination of two or more. Among these, amides are preferable, and N,N-dimethylacetamide and N,N-dimethylformamide are particularly preferable.

The amount of the solvent used is preferably 50 to 500 parts by mass, more preferably 70 to 400 parts by mass, and 75 to 250 parts by mass relative to the total amount of the ester-type epoxy furan resin and the dicarboxylic acid compound being 100 parts by mass.

### [Ester-type epoxy furan resin composition]

The ester-type epoxy furan resin composition of the present invention (hereinafter also simply referred to as a resin composition) contains the above furan resin and further contains preferably at least any of an epoxy curing agent and a resin other than the furan resin of the present invention.

Here, a known curing agent capable of curing an epoxy resin can be used as the epoxy curing agent. Specific examples of the epoxy curing agent include imidazole curing agents such as 2-methylimidazole, 2-ethylimidazole, 2-undecylimidazole, 2,4-dimethylimidazole, 2-heptadecylimidazole, 1,2-dimethylimidazole, 1,2-diethylimidazole, 2-phenyl-4-methylimidazole, 2,4,5-triphenylimidazole, 2-ethyl-4-methylimidazole, 2-phenylimidazole, 1-benzyl-2-phenylimidazole, 1-cyanoethyl-2-methylimidazole, 1-cyanoethyl-2-undecylimidazole, 1-benzyl-2-methylimidazole, 2-phenyl-4,5-dihydroxymethylimidazole, 2-aryl-4,5-diphenylimidazole, 2,4-diamino-6-[2'-methylimidazolyl-(1)']-ethyl-S-triazine, 2,4-diamino-6-[2'-ethyl-4'-methylimidazolyl-(1)']-ethyl-S-triazine, 2,4-diamino-6-[2'-methylimidazolyl-(1)']-ethyl-S-triazine isocyanuric acid adduct, and 2-phenyl-4-methyl-5-hydroxymethylimidazole; amine curing agents such as ethylenediamine, diethylenetriamine, triethylenetetramine, tetraethylenepentamine, dipropylenetriamine, diethylaminopropylamine, N-aminoethylpiperazine, isophoronediamine, bis(4-amino-3-methylcyclohexyl)methane, menthanediamine, 1,3-bisaminomethylcyclohexane, xylylenediamine, phenylenediamine, diaminodiphenylmethane, and diaminodiphenylsulfone; acid anhydride curing agents such as phthalic anhydride, tetrahydrophthalic anhydride, hexahydrophthalic anhydride, trimellitic anhydride, pyromellitic anhydride, benzophenonetetracarboxylic anhydride, chlorendic anhydride, dodecenylsuccinic anhydride, methyltetrahydrophthalic anhydride, methylendomethylenetetrahydrophthalic anhydride, and methylhexahydrophthalic anhydride represented by 4-methylhexahydrophthalic anhydride; polyamide resins that are condensates of dimer or trimer acids and polyamines; Lewis acids such as boron trifluoride-amine complex; amideamine curing agents such as dicyandiamide; and phenol or derivatives thereof. Among these, it is preferable to use an imidazole curing agent or an acid anhydride curing agent, and, in particular, it is more preferable to use an imidazole curing agent and an acid anhydride curing agent in combination. The use of these curing agents in combination is likely to promote the curing of the resin composition.

In the resin composition containing an epoxy curing agent, the furan resin normally constitutes the base compound of resin components, and the resin composition is formed into a cured resin product by crosslinking or curing the base-compound furan resin with the epoxy curing agent. The content of the epoxy curing agent in the resin composition is suitably adjusted according to the epoxy equivalent of the furan resin and is, for example, 1 to 100 parts by mass and preferably 2 to 50 parts by mass relative to 100 parts by mass of the furan resin.

A further resin other than the furan resin may be contained in the resin composition as described above. Examples of such resins include acrylic resins, polyester resins, polyamide resins, polyurethane resins, phenol resins, and epoxy resins other than the furan resin of the present invention. These resins are preferably polymers having a weight average molecular weight of 10,000 to 1,000,000. The weight average molecular weight is a value obtained by, for example, gel permeation chromatography (GPC) in terms of polystyrene.

These further resins may or may not contain a reactive functional group capable of reacting with the epoxy group contained in the furan resin, such as an amino group, a carboxyl group, a carboxylic anhydride group, or a phenolic hydroxy group. When the reactive functional group is contained, the furan resin of the present invention reacts with the above resins and is used as a crosslinking agent. Naturally, the resin composition may contain the above epoxy curing agent in addition to the furan resin and the further resins.

The furan resin is contained preferably in an amount of about 0.1 to 100 parts by mass, more preferably about 0.5 to 50 parts by mass, and even more preferably about 1 to 30 parts by mass relative to 100 parts by mass of these further resins.

The resin composition may contain a coupling agent. The coupling agent is suitably used when the resin composition constitutes a cured composite which will be described below. The coupling agent can improve adhesion between a fibrous base material and a cured resin product.

The coupling agent is preferably a silane coupling agent. Examples of the silane coupling agent include silane coupling agents such as 2-(3,4-epoxycyclohexyl)ethyltrimethoxysilane, 3-glycidoxypropylmethyldimethoxysilane, 3-glycidoxypropyltrimethoxysilane, 3-glycidoxypropylmethyldiethoxysilane, 3-glycidoxypropyltriethoxysilane, N-2-(aminoethyl)-3-aminopropylmethyldimethoxysilane, N-2-(aminoethyl)-3-aminopropyltrimethoxysilane, 3-aminopropyltrimethoxysilane, 3-aminopropyltriethoxysilane, 3-triethoxysilyl-N-(1,3-dimethylbutylidene)propylamine, N-phenyl-3-aminopropyltrimethoxysilane, N-(vinylbenzyl)-2-aminoethyl-3-aminopropyltrimethoxysilane hydrochloride, tris-(trimethoxysilylpropyl)isocyanurate, 3-ureidopropyltrialkoxysilane, 3-mercaptopropylmethyldimethoxysilane, 3-mercaptopropyltrimethoxysilane, bis(triethoxysilylpropyl)tetrasulfide, and 3-isocyanatepropyltriethoxysilane. Among these, silane coupling agents containing an epoxy group are preferable, such as 3-glycidoxypropyltrimethoxysilane.

The content of the coupling agent in the resin composition is preferably 0.1 to 5 parts by mass and more preferably 0.3 to 3 parts by mass relative to 100 parts by mass of the furan resin.

The resin composition may further contain additives that are used in conventional epoxy resins. Examples of such additives include fillers, plasticizers, and pigments.

### [Cured resin product]

The cured resin product of the present invention is a cured product of the above resin composition, and has superior mechanical strength and extensibility. The cured resin product of the present invention is obtained by, for example, heat-curing the above resin composition at 15 to 200°C and preferably 50 to 190°C.

As for a specific curing method, the resin composition can be cured by placing the resin composition in a container or a mold having a predetermined shape and heating the resin composition in a thermostatic chamber or a thermostatic bath adjusted to the above temperature. The resin composition can also be cured by circulating hot air or hot water adjusted to the above temperature through a container or a mold having a predetermined shape.

The cured resin product of the present invention may constitute a cured composite together with a fibrous substrate by impregnating the fibrous substrate with the resin composition and curing the resin composition. The method for curing the resin composition with which a fibrous substrate is impregnated is not particularly limited, and examples include a method for heat-curing the fibrous substrate impregnated with the composition placed in a mold with hot air or between hot plates.

Examples of the fibrous substrate include a fabric, a nonwoven fabric of glass fiber, carbon fiber, metal fiber, paper, cotton, hemp, etc, a chopped strand mat, and a roving cloth.

As the raw material for the nonwoven fabric, for example, polyester, high-density polyethylene (HDPE), polypropylene, and the like are preferred. Alternatively, a felt, a mat, a spun bonded fabric, a web, or the like which is a porous material with flexibility, having continuous filaments or staple fibers, may be used. The chopped strand mat is produced, for example, preferably by cutting strands of glass fiber or the like into a predetermined length to be dispersed in a mat form, and then uniformly applying an adhesive of a thermoplastic resin or the like to be thermally melted such that the strands are adhered to each other to form a mat. The roving cloth is preferably formed of reinforced fiber such as glass fiber, carbon fiber, aramid fiber, inorganic fiber, organic fiber, or whisker.

The furan resin of the present invention can be used in various fields such as electric fields, civil engineering fields, construction fields, and machinery fields, and, for example, can be used for steel-pipe lining materials, joint cement, adhesives, FRPs, prepregs, etc.

As described above, the furan resin of the present invention has a furan ring as its backbone, and thus can be easily synthesized from renewable resources. Moreover, the furan resin of the present invention has a relatively well-defined structure, and thus can be easily produced to have a desired structure by the above production method. Furthermore, a cured resin product produced from the furan resin can have superior physical properties such as mechanical strength, extensibility, and adhesion.

### Examples

The following examples are provided to illustrate the present invention but are not intended to limit the scope of the invention. The conditions of each measurement in the following examples are as follows.

### <Measurement conditions>

### [GPC measurement]

A GPC system manufactured by Shimadzu Corporation with columns "KF803L" x2 manufactured by Showa Denko K.K. (exclusion limit molecular weight: 70,000) was used. The column temperature was set at 40°C, and the flow rate was set at 1.0 ml/min. Tetrahydrofuran was used as an eluent, and an RI was used as a detector.

In the method for calculating a molecular weight, a calibration curve was made using standard polystyrenes "TSK standard POLYSTYLENE" having weight average molecular weights (Mw) of 96,400, 37,900, 18,100, 9,100, 5,970, 2,630, 1,050, and 500, respectively. The weight average molecular weight (Mw), the number average molecular weight (Mn), and the molecular weight distribution (Mw/Mn) were obtained by calculation.

### [Measurement of ¹H-NMR spectrum]

Using an NMR measurement apparatus "ECX-400" manufactured by JEOL Limited, the measurement was performed at 23°C in a solvent of deuterated chloroform or deuterated DMSO.

### [FT-IR measurement]

The measurement was performed by an ATR method using "NICOLET 380" manufactured by Thermo Electron as a spectrometer.

### Example 1

As shown in the following reaction formula, 100 parts by mass of bisfurandicarboxylic acid (A), 699 parts by mass of epichlorohydrin (B), and 2.4 parts by mass of tetra-n-butylammonium bromide as a catalyst were charged into a flask and reacted for 1 hour by being heated to 90°C. Subsequently, 90.8 parts by mass of a 40 mass% aqueous sodium hydroxide solution was slowly added, after addition the reaction was allowed to proceed for 1 more hour, and after the completion of the reaction, the resulting reaction solution was cooled down, and precipitates were filtered off from the reaction solution. Subsequently, the reaction solution was diluted with 680 parts by mass of ethyl acetate and washed with saturated brine 3 times to remove the aqueous-phase part, and then the reaction solution was dried over magnesium sulfate. Then, ethyl acetate and an excess of epichlorohydrin were distilled off under reduced pressure, and the intended-product ester-type epoxy furan resin was thus obtained. The verification of the structure by ¹H-NMR and FT-IR resulted in the ester-type epoxy furan resin (C) shown in the following reaction formula. Furthermore, the measurement by the GPC method of the molecular weight (in terms of standard polystyrene) of the resulting ester-type epoxy furan resin resulted in a number average molecular weight Mn of 610, a weight average molecular weight Mw of 780, and a molecular weight distribution Mw/Mn of 1.27. Accordingly, the average value of n calculated from Mn was 0.73.

The ester-type epoxy furan resin obtained above was purified with an ethyl acetate/hexane mixed solvent as a developer solvent using a silica gel column to isolate a compound having n = 0. The ¹H-NMR spectrum of the resulting compound in heavy chloroform was as follows.
δ 1.74 (s, 6H), 2,71 (dd, 2H), 2.88 (t, 2H), 3.30 (m, 2H), 4.11 (dd, 2H), 4.60 (dd, 2H), 6.22 (d, 2H), 7.15 (d, 2H)

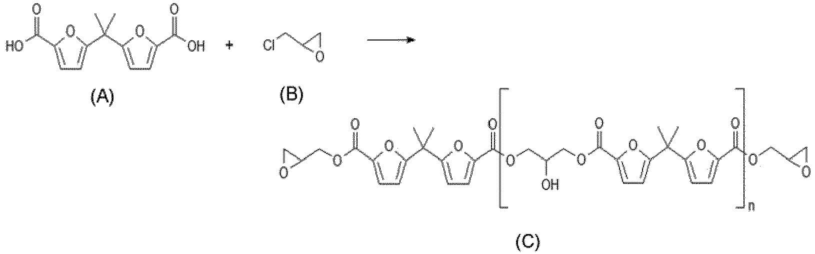

### Example 2

100 parts by mass of the ester-type epoxy furan resin obtained in Example 1 was mixed with 44.7 parts by mass of 4-methylhexahydrophthalic anhydride and 0.7 parts by mass of 2-ethyl-4-methylimidazole, and the mixture was preliminary reacted by being heated to 100°C, and then cast onto a PET film by a doctor blade. This film was heat-treated at 120°C, 150°C, and 180°C each for 1 hour to thereby prepare cured films having a thickness of 140 µm. The evaluation of the breaking strength and the breaking elongation of the resulting films by the evaluation method which will be described below all resulted in "A", indicating superior breaking strength and breaking elongation.

### Example 3

An ester-type epoxy furan resin was synthesized as in Example 1 except that the heating temperature during reaction was 95°C. The verification of the structure by ¹H-NMR and FT-IR in the same manner as Example 1 resulted in the ester-type epoxy furan resin (C) shown in the above reaction formula. The measurement by the GPC method of the molecular weight (in terms of standard polystyrene) of the resulting ester-type epoxy furan resin resulted in a number average molecular weight Mn of 680, a weight average molecular weight Mw of 1030, and a molecular weight distribution Mw/Mn of 1.51. Accordingly, the average value of n calculated from Mn was 0.95.

### Example 4

100 parts by mass of the ester-type epoxy furan resin obtained in Example 3 was mixed with 25 parts by mass of 4-methylhexahydrophthalic anhydride, 0.63 parts by mass of 2-ethyl-4-methylimidazole, and 1.25 parts by mass of 3-glycidoxypropyltrimethoxysilane, and glass fiber cloth was impregnated with the mixture. This was heat-treated at 120°C, 150°C, and 180°C each for 1 hour to thereby prepare cured composite sheets having a thickness of 0.3 mm. The evaluation of the breaking strength and the breaking elongation of the resulting cured composite sheets by the evaluation method which will be described below all resulted in "A", indicating superior breaking strength and breaking elongation.

### Example 5

### (Chain extending reaction)

100 parts by mass of the ester-type epoxy furan resin obtained in Example 3, 32.5 parts by mass of bisfurandicarboxylic acid (0.84 mol relative to the furan resin), and 150 parts by mass of N,N-dimethylacetamide were mixed to form a uniform solution and reacted for 3 hours by being heated to 130°C. The reacted solution was cooled and then introduced into large amounts of methanol to precipitate and isolate the resin, and the remaining solvent was distilled off under reduced pressure. The verification of the structure of the resulting resin by ¹H-NMR measurement in deuterated chloroform resulted in the structure of the general formula (3). The measurement by the GPC method of the molecular weight (in terms of standard polystyrene) of the resulting resin resulted in a number average molecular weight Mn of 6070, a weight average molecular weight Mw of 12650, and a molecular weight distribution Mw/Mn of 2.08. Accordingly, the average value of n calculated from Mn was 17.8.

### Example 6

In accordance with the above reaction formula, 100 parts by mass of bisfurandicarboxylic acid (A), 1000 parts by mass of epichlorohydrin (B), and 7.3 parts by mass of benzyltriethylammonium chloride as a catalyst were charged into a flask and reacted for 1 hour by being heated to 90°C. Subsequently, 102.5 parts by mass of a 50 mass% aqueous sodium hydroxide solution was slowly added, after addition, the reaction was allowed to proceed for 2 more hours, and after the completion of the reaction, cooling was performed to filter off precipitates from the reaction solution. Subsequently, the reaction solution was washed with an aqueous sodium hydrogencarbonate solution and ion exchanged water until becoming neutral to remove the aqueous-phase part, and the reaction solution was dried over magnesium sulfate. Then, an excess of epichlorohydrin was distilled off under reduced pressure to obtain the intended product.

The structure of the intended product was verified by ¹H-NMR and FT-IR in the same manner as Example 1, and the resulting intended product was the ester-type epoxy furan resin (C) shown in the above reaction formula.

### [Evaluation method]

### <Cured film>

The cured films cut into strips having a width of 10 mm were subjected to a tensile test at a tensile rate of 2 mm/min with a tensile tester "Autograph AG-5000B" manufactured by Shimadzu Corporation to measure breaking strength and breaking elongation, and evaluated according to the following criteria.

### (Breaking strength)

| | |
|---|---|
| 40 MPa or more: A (Breaking elongation) | Less than 40 MPa: B |
| 2% or more: A | Less than 2%: B |

### <Cured composite sheet>

The cured composite sheets cut into strips having a width of 10 mm were subjected to a tensile test at a tensile rate of 2 mm/min with a tensile tester "Autograph AG-5000B" manufactured by Shimadzu Corporation to measure breaking strength and breaking elongation, and evaluated according to the following criteria.

### (Breaking strength)

| | |
|---|---|
| 140 MPa or more: A (Breaking elongation) | Less than 140 MPa: B |
| 2% or more: A | Less than 2%: B |

## Claims

1. An ester-type epoxy furan resin represented by the following general formula (1): 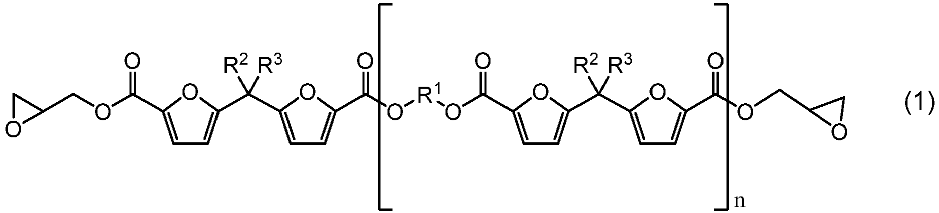 wherein R¹ is an alkylene group having 2 to 6 carbon atoms optionally containing a hydroxy group; R² and R³ are each independently an alkyl group having 1 to 4 carbon atoms, and R² and R³ may be bonded to each other to form a cyclic structure; n is 0 to 300; and a plurality of R¹, R², and R³, if present within a molecule, may be mutually the same or different.

2. The ester-type epoxy furan resin according to claim 1, represented by the following general formula (2): 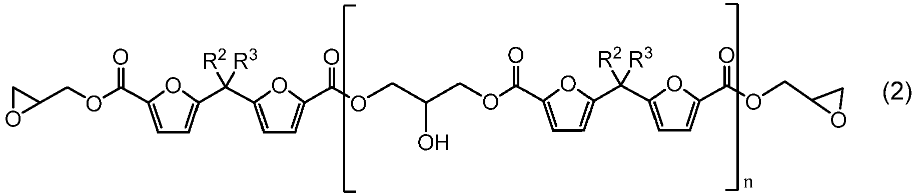 wherein R², R³, and n are the same as above.

3. The ester-type epoxy furan resin according to claim 2, represented by the following general formula (3): 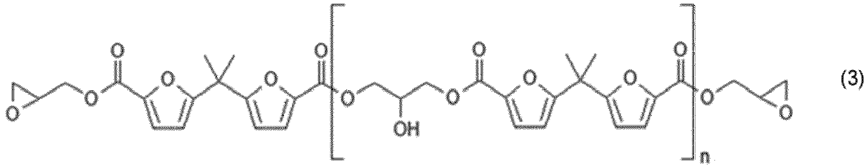 wherein n is the same as above.

4. The ester-type epoxy furan resin according to any one of claims 1 to 3, derived from a renewable resource.

5. A method for producing the ester-type epoxy furan resin according to any one of claims 1 to 4, comprising reacting a dicarboxylic acid compound represented by the following general formula (4) with epihalohydrin: 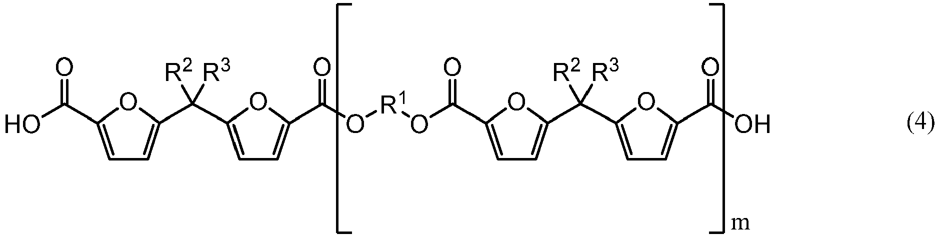 wherein R¹, R², and R³ are the same as above; and m is 0 to 300.

6. A method for producing the ester-type epoxy furan resin according to any one of claims 1 to 4, comprising:
reacting an ester-type epoxy furan resin having a structure represented by the general formula (1) wherein n is less than that of an ester-type epoxy furan resin to be obtained with a dicarboxylic acid compound represented by the following general formula (4-1): 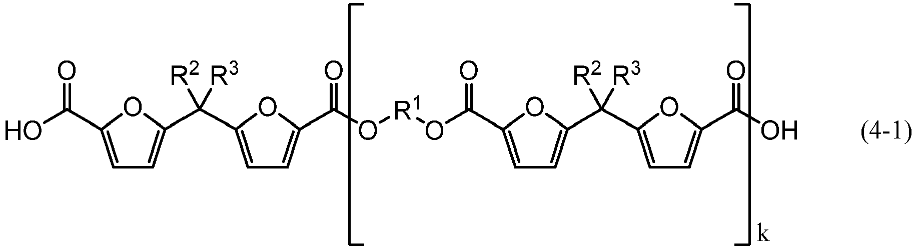
wherein R¹, R², and R³ are the same as above; and k is 0 or more and less than 300,
to obtain an ester-type epoxy furan resin represented by the general formula (1).

7. The method for producing an ester-type epoxy furan resin according to claim 5 or 6, wherein the dicarboxylic acid compound is a bisfurandicarboxylic acid compound represented by the following general formula (5): 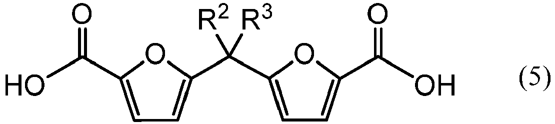 wherein R² and R³ are the same as above.

8. The method for producing an ester-type epoxy furan resin according to any one of claims 5 to 7, wherein the dicarboxylic acid compound is derived from a renewable resource.

9. A resin composition comprising the ester-type epoxy furan resin according to any one of claims 1 to 4 and at least any of an epoxy curing agent and a resin other than the ester-type epoxy furan resin.

10. A cured resin product, obtained by curing the resin composition according to claim 9.

## Amended claims

### Amended claims under Art. 19.1 PCT

**1.** An ester-type epoxy furan resin represented by the following general formula (1): 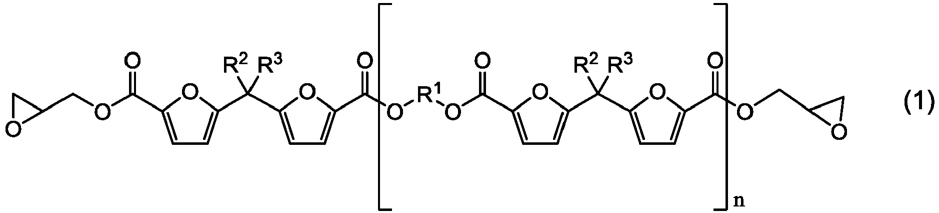 wherein R¹ is an alkylene group having 2 to 6 carbon atoms optionally containing a hydroxy group; R² and R³ are each independently an alkyl group having 1 to 4 carbon atoms, and R² and R³ may be bonded to each other to form a cyclic structure; n is 0 to 300; and a plurality of R¹, R², and R³, if present within a molecule, may be mutually the same or different.

**2.** The ester-type epoxy furan resin according to claim 1, represented by the following general formula (2): 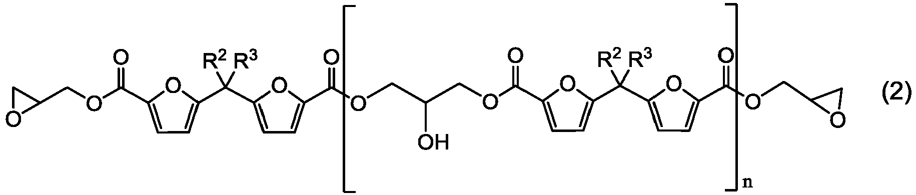 wherein R², R³, and n are the same as above.

**3.** The ester-type epoxy furan resin according to claim 2, represented by the following general formula (3): 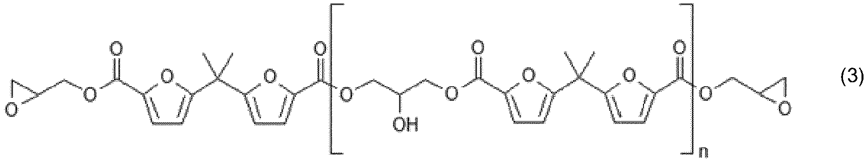 wherein n is the same as above.

**4.** The ester-type epoxy furan resin according to any one of claims 1 to 3, derived from a renewable resource.

**5.** (Amended) The ester-type epoxy furan resin according to any one of claims 1 to 4, having a weight average molecular weight (Mw) of 400 to 8000.

**6.** (Amended) A method for producing the ester-type epoxy furan resin according to any one of claims 1 to 5, comprising reacting a dicarboxylic acid compound represented by the following general formula (4) with epihalohydrin: 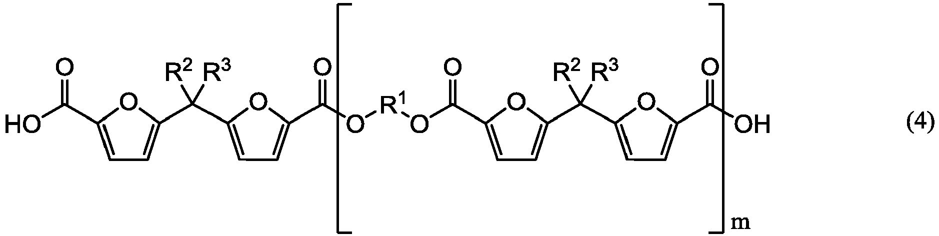 wherein R¹, R², and R³ are the same as above; and m is 0 to 300.

**7.** (Amended) The method for producing an ester-type epoxy furan resin according to claim 6, wherein the reaction is carried out in the presence of a catalyst and an alkali compound, and the catalyst is a quaternary ammonium salt.

**8.** (Amended) A method for producing the ester-type epoxy furan resin according to any one of claims 1 to 5, comprising:
reacting an ester-type epoxy furan resin having a structure represented by the general formula (1) wherein n is less than that of an ester-type epoxy furan resin to be obtained with a dicarboxylic acid compound represented by the following general formula (4-1): 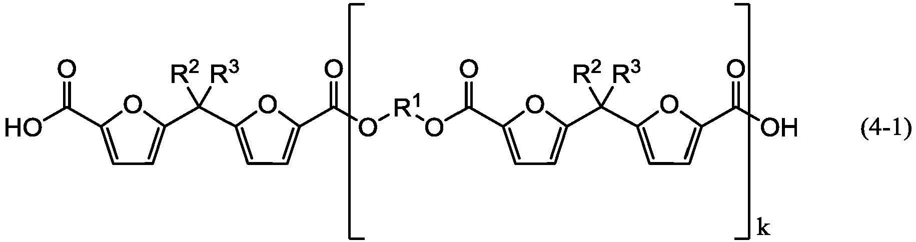
wherein R¹, R², and R³ are the same as above; and k is 0 or more and less than 300,
to obtain an ester-type epoxy furan resin represented by the general formula (1).

**9.** (Amended) The method for producing an ester-type epoxy furan resin according to any one of claims 6 to 8, wherein the dicarboxylic acid compound is a bisfurandicarboxylic acid compound represented by the following general formula (5): 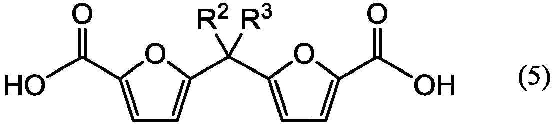 wherein R² and R³ are the same as above.

**10.** (Amended) The method for producing an ester-type epoxy furan resin according to any one of claims 6 to 9, wherein the dicarboxylic acid compound is derived from a renewable resource.

**11.** Added) A resin composition comprising the ester-type epoxy furan resin according to any one of claims 1 to 5 and at least any of an epoxy curing agent and a resin other than the ester-type epoxy furan resin.

**12.** Added) The resin composition according to claim 11, comprising the ester-type epoxy furan resin and the epoxy curing agent, wherein the epoxy curing agent comprises an imidazole curing agent and an acid anhydride curing agent.

**13.** Added) The resin composition according to claim 11 or 12, further comprising a coupling agent, wherein the coupling agent is a silane coupling agent.

**14.** Added) The resin composition according to claim 13, wherein the silane coupling agent is a silane coupling agent containing an epoxy group.

**15.** Added) A cured resin product, obtained by curing the resin composition according to any one of claims 11 to 14.
